# EUROPEAN PATENT APPLICATION

(11) **EP 4 112 639 A1**
(43) Date of publication of application: **04.01.2023**
(21) Application number: 21382593.8
(22) Date of filing: 02.07.2021
(51) Int. Cl.: C07K 14/725, A61K 35/17, C07K 14/47, C07K 14/705

(54) **CAR T/NK-CELLS FOR USE IN THE TREATMENT OF INVASIVE FUNGAL INFECTIONS**

(71) Applicant: Universitat de Barcelona, 08028 Barcelona (ES); Hospital Clínic de Barcelona, 08036 Barcelona (ES); Institut d'Investigacions Biomèdiques August Pi i Sunyer (IDIBAPS), 08036 Barcelona (ES)
(72) Inventor: LOZANO SOTO, Francisco, 08036 Barcelona (ES); VELASCO DE ANDRÉS, María, 08036 Barcelona (ES)
(74) Representative: Hoffmann Eitle

(57) **Abstract**

**INFECTIONS.** The present invention refers to the medical field. Particularly, the present invention refers to autologous/allogeneic CAR (chimeric antigen receptor)-T/NK cells for use in the treatment of invasive fungal infections.

## Description

### FIELD OF THE INVENTION

The present invention refers to the medical field. Particularly, the present invention refers to CAR (chimeric antigen receptor) T/NK-cells for use in the treatment of fungal infections.

### STATE OF THE ART

Fungi are ubiquitous environmental organisms with special clinical relevance in immunocompromised individuals and hospitalized patients. The spectrum of fungal diseases in humans ranges from mild skin or mucosal infections to life-threatening invasive fungal infections (IFIs). Mortality and morbidity rates due to fungal infections are still rising as a consequence of multiple factors, comprising: (a) the increasing number of surgical procedures and subsequent augment of intensive care units (ICU) admitted patients, (b) the emergence of multidrug resistant (MDR) pathogens, (c) clinical interventions triggering neutropenia, (d) the AIDS epidemics, and (e) population aging.

Current antifungal treatment effectiveness depends on drug selection and diagnosis. In this context, azoles, echinocandins and polyenes are the main antifungal agents used for IFIs treatment. More specifically, ergosterol and 1,3-β-glucan biosynthesis inhibitors, such as fluconazole and caspofungin, respectively, are used as first-line therapeutic agents.

However, these compounds have multiple side effects including cross-resistance, toxicity and drug interactions.

So, there is an unmet medical need of finding alternative antifungal therapies, which can be used to efficiently treat fungal infection without causing relevant side effects.

The present invention is focused on solving this problem and a new antifungal therapy is herein in proposed, which is based on the adoptive cell transfer of autologous Tαβ or allogeneic Tγδ or NK cells expressing a specific CAR.

### DESCRIPTION OF THE INVENTION

As explained above, the present invention refers to autologous/allogeneic CAR T/NK-cells, comprising a specific CAR, for use in the treatment of fungal infections.

Particularly, the inventors of the present invention have developed specific CAR T/NK-cells for adoptive cell immunotherapy to severe fungal infections. The autologous/allogeneic CAR T/NK cells of the present invention have been made by transducing these cells with a lentiviral vector comprising a nucleic acid encoding SEQ ID NO: 1.

Such as it can be seen in the Examples provided below, these CAR T/NK cells have been tested *in vitro* and *in vivo,* showing an improved survival of mice infected with *Candida albicans.* So, the effect of the CAR T/NK over C. *albicans* is herein used as proof of concept, but this therapy could be efficiently used against other invasive fungal infections, preferably caused by fungal species from the genus *Candida, Aspergillus, Fusarium, Cryptococcus, Paracoccidiodes, Histoplasma, Rizhopus, Mucor* or *Arthrocladium.*

So, the first embodiment of the present invention refers to a CAR (hereinafter CAR of the invention) comprising an extracellular domain which (in turn) comprises SEQ ID NO: 1. In other words, the extracellular domain of the CAR of the invention is characterized by comprising SEQ ID NO: 1.

In a preferred embodiment, the CAR of the invention comprises an extracellular domain which consists of the SEQ ID NO: 1 or SEQ ID NO: 2. So, in a preferred embodiment, the extracellular domain of the CAR of the invention is characterized by consisting of SEQ ID NO: 1 or SEQ ID NO: 2.

SEQ ID NO: 1 is as follows:

RLSWYDPDFQARLTRSNSKCQGQLEVYLKDGWHMVCSQSWGRSSKQWEDPSQAS
KVCQRLNCGVPLSLGPFLVTYTPQSSIICYGQLGSFSNCSHSRNDMCHSLGLTCLEPQ
KTTPPTTRPPPTTTPEPTAPPRLQLVAQSGGQHCAGVVEFYSGSLGGTISYEAQDKTQ
DLENFLCNNLQCGSFLKHLPETEAGRAQDP/LGEPREHQPLPIQWKIQNSSCTSLEHCF
RKIKPQKSGRVLALLCSGFQPKVQSRLVGGSSICEGTVEVRQGAQWAALCDSSSARS
SLRWEEVCREQQCGSVNSYRVLDAGDPTSRGLFCPHQKLSQCHELWERNSYCKKVF
VTCQD

*Note: P (220) is polymorphic in human CD5 and can be replaced by L.*

SEQ ID NO: 1 corresponds with the position R25 to D369 of the whole CD5 extracellular sequence (SEQ ID NO: 2). The sequence of the whole CD5 extracellular (SEQ ID NO: 2) is depicted below, wherein the SEQ ID NO: 1 is underlined:

MPMGSLQPLATLYLLGMLVASCLGRLSWYDPDFQARLTRSNSKCQGQLEVYLKDG
WHMVCSQSWGRSSKQWEDPSQASKVCQRLNCGVPLSLGPFLVTYTPQSSIICYGQL
GSFSNCSHSRNDMCHSLGLTCLEPQKTTPPTTRPPPTTTPEPTAPPRLQLVAQSGGQH
CAGVVEFYSGSLGGTISYEAQDKTQDLENFLCNNLQCGSFLKHLPETEAGRAQDP/L
GEPREHQPLPIQWKIQNSSCTSLEHCFRKIKPQKSGRVLALLCSGFQPKVQSRLVGGS
SICEGTVEVRQGAQWAALCDSSSARSSLRWEEVCREQQCGSVNSYRVLDAGDPTSR
GLFCPHQKLSQCHELWERNSYCKKVFVTCQDPNP

*Note: P (224) is polymorphic in human CD5 and can be replaced by L.*

In a preferred embodiment, the CAR of the invention further comprises a signal peptide, a transmembrane domain, and one or more intracellular activation domains.

In a preferred embodiment, the signal peptide consist of CD8α of SEQ ID NO: 3, the hinge domain consists of CD8α of SEQ ID NO: 4, the transmembrane domain consists of CD8α of SEQ ID NO: 5 or NKGD2 of SEQ ID NO: 6, and the intracellular activation domain consists of CD137/4-1BB of SEQ ID NO: 7, CD28 of SEQ ID NO: 8, CD244/2B4 of SEQ ID NO: 9 and/or of CD3ζ (zeta) of SEQ ID NO: 10.

SEQ ID NO: 3 of the CD8α signal peptide is as follows:
MALPVTGLLLSLGLLLHAARP

SEQ ID NO: 4 of the CD8α hinge region is as follows:
TTTPAPRPPTPAPTIASQPLSLRPEACRPAAGGAVHTRGLDFACDI

SEQ ID NO: 5 of the CD8α transmembrane region is as follows:
YIWAPLAGTCGVLLLSLVITLYC

SEQ ID NO: 6 of the NKG2D transmembrane domain is as follows:
PFFFCCFIAVAMGIRFIIMVAIW

SEQ ID NO: 7 of the CD137/4-1BB intracellular activation domain is as follows:
KRGRKKLLYIFKQPFMRPVQTTQEEDGCSCRFPEEEEGGCEL

SEQ ID NO: 8 of the CD28 intracellular activation domain is as follows:
RSKRSRLLHSDYMNMTPRRPGPTRKHYQPYAPPRDFAAYRS

SEQ ID NO: 9 of the CD244/2B4 intracellular activation domain as follows:

WRRKRKEKQSETSPKEFLTIYEDVKDLKTRRNHEQEQTFPGGGSTIYSMIQSQSSAPT
SQEPAYTLYSLIQPSRKSGSRKRNHSPSFNSTIYEVIGKSQPKAQNPARLSRKELENFD
VYS

SEQ ID NO: 10 of the CD3ζ (zeta) intracellular activation domain is as follows:

RVKFSRSADAPAYQQGQNQLYNELNLGRREEYDVLDKRRGRDPEMGGKPQRRKNP
QEGLYNELQKDKMAEAYSEIGMKGERRRGKGHDGLYQGLSTATKDTYDALHMQA
LPPR

In a preferred embodiment, the CAR of the invention comprises or consists of SEQ ID NO: 11, SEQ ID NO: 12 or SEQ ID NO: 13. So, three versions of the CAR of the invention have been designed in the context of the present invention:
- Variant 1 corresponds with SEQ ID NO: 11 which comprises: CD8α signal peptide, CD5 extracellular region, CD8α hinge and transmembrane, CD137/4-1BB intracellular activation domain and CD3ζ intracellular activation domain.

SEQ ID NO: 11 is as follows:

MALPVTGLLLSLGLLLHAARPRLSWYDPDFQARLTRSNSKCQGQLEVYLKDGWHM
VCSQSWGRSSKQWEDPSQASKVCQRLNCGVPLSLGPFLVTYTPQSSIICYGQLGSFSN
CSHSRNDMCHSLGLTCLEPQKTTPPTTRPPPTTTPEPTAPPRLQLVAQSGGQHCAGVV
EFYSGSLGGTISYEAQDKTQDLENFLCNNLQCGSFLKHLPETEAGRAQDP/LGEPREH
QPLPIQWKIQNSSCTSLEHCFRKIKPQKSGRVLALLCSGFQPKVQSRLVGGSSICEGTV
EVRQGAQWAALCDSSSARSSLRWEEVCREQQCGSVNSYRVLDAGDPTSRGLFCPHQ
KLSQCHELWERNSYCKKVFVTCQDTTTPAPRPPTPAPTIASQPLSLRPEACRPAAGGA
VHTRGLDFACDIYIWAPLAGTCGVLLLSLVITLYCKRGRKKLLYIFKQPFMRPVQTT
QEEDGCSCRFPEEEEGGCELRVKFSRSADAPAYQQGQNQLYNELNLGRREEYDVLD
KRRGRDPEMGGKPQRRKNPQEGLYNELQKDKMAEAYSEIGMKGERRRGKGHDGL
YQGLSTATKDTYDALHMQALPPR

*Note: P (221) is polymorphic in human CD5 and can be replaced by L.*
- Variant 2 of the CAR of the invention corresponds with SEQ ID NO: 12 which comprises: CD8α signal peptide, CD5 extracellular region, CD8α hinge and transmembrane region, CD28 intracellular activation domain, CD137/4-1BB intracellular activation domain and CD3ζ intracellular activation domain.

SEQ ID NO: 12 is as follows:

MALPVTGLLLSLGLLLHAARPRLSWYDPDFQARLTRSNSKCQGQLEVYLKDGWHM
VCSQSWGRSSKQWEDPSQASKVCQRLNCGVPLSLGPFLVTYTPQSSIICYGQLGSFSN
CSHSRNDMCHSLGLTCLEPQKTTPPTTRPPPTTTPEPTAPPRLQLVAQSGGQHCAGVV
EFYSGSLGGTISYEAQDKTQDLENFLCNNLQCGSFLKHLPETEAGRAQDP/LGEPREH
QPLPIQWKIQNSSCTSLEHCFRKIKPQKSGRVLALLCSGFQPKVQSRLVGGSSICEGTV
EVRQGAQWAALCDSSSARSSLRWEEVCREQQCGSVNSYRVLDAGDPTSRGLFCPHQ
KLSQCHELWERNSYCKKVFVTCQDTTTPAPRPPTPAPTIASQPLSLRPEACRPAAGGA
VHTRGLDFACDIYIWAPLAGTCGVLLLSLVITLYCRSKRSRLLHSDYMNMTPRRPGP
TRKHYQPYAPPRDFAAYRSKRGRKKLLYIFKQPFMRPVQTTQEEDGCSCRFPEEEEG
GCELRVKFSRSADAPAYQQGQNQLYNELNLGRREEYDVLDKRRGRDPEMGGKPQR
RKNPQEGLYNELQKDKMAEAYSEIGMKGERRRGKGHDGLYQGLSTATKDTYDALH
MQALPPR

*Note: P (221) is polymorphic in human CD5 and can be replaced by L.*
- Variant 3 of the CAR of the invention corresponds with SEQ ID NO: 13 which comprises: CD8α signal peptide, CD5 extracellular region, CD8αhinge region, NKG2D transmembrane domain, CD244/2B4 intracellular activation domain and CD3ζ intracellular activation domain.

SEQ ID NO: 13 is as follows:

MALPVTGLLLSLGLLLHAARPRLSWYDPDFQARLTRSNSKCQGQLEVYLKDGWHM
VCSQSWGRSSKQWEDPSQASKVCQRLNCGVPLSLGPFLVTYTPQSSIICYGQLGSFSN
CSHSRNDMCHSLGLTCLEPQKTTPPTTRPPPTTTPEPTAPPRLQLVAQSGGQHCAGVV
EFYSGSLGGTISYEAQDKTQDLENFLCNNLQCGSFLKHLPETEAGRAQDP/LGEPREH
QPLPIQWKIQNSSCTSLEHCFRKIKPQKSGRVLALLCSGFQPKVQSRLVGGSSICEGTV

EVRQGAQWAALCDSSSARSSLRWEEVCREQQCGSVNSYRVLDAGDPTSRGLFCPHQ
KLSQCHELWERNSYCKKVFVTCQDTTTPAPRPPTPAPTIASQPLSLRPEACRPAAGGA
VHTRGLDFACDIPFFFCCFIAVAMGIRFIIMVAIWWRRKRKEKQSETSPKEFLTIYEDV
KDLKTRRNHEQEQTFPGGGSTIYSMIQSQSSAPTSQEPAYTLYSLIQPSRKSGSRKRN
HSPSFNSTIYEVIGKSQPKAQNPARLSRKELENFDVYSRVKFSRSADAPAYQQGQNQ
LYNELNLGRREEYDVLDKRRGRDPEMGGKPQRRKNPQEGLYNELQKDKMAEAYSE
IGMKGERRRGKGHDGLYQGLSTATKDTYDALHMQALPPR

*Note: P (221) is polymorphic in human CD5 and can be replaced by L.*

The second embodiment of the present invention refers to a nucleic acid encoding the CAR of the invention.

In a preferred embodiment, the nucleic acid comprises or consists of SEQ ID NO: 14, SEQ ID NO: 15 or SEQ ID NO: 16.

SEQ ID NO: 14 is the nucleotide sequence which encodes CAR variant 1. SEQ ID NO: 14 is as follows:

ATGGCCTTACCAGTGACCGGCTTGCTCCTGTCGCTGGGCTTGCTGCTCCACGCCG
CCAGGCCGAGACTGAGTTGGTACGATCCCGACTTCCAGGCACGACTGACACGGA
GTAATAGTAAATGCCAGGGGCAGCTGGAGGTGTATCTGAAAGACGGATGGCACA
TGGTGTGCAGTCAGTCATGGGGCCGGAGCTCCAAGCAGTGGGAGGATCCTTCCC
AGGCCTCTAAAGTGTGCCAGAGACTGAACTGTGGAGTCCCTCTGTCCCTGGGCCC
ATTCCTGGTCACATACACTCCACAGTCTAGTATCATTTGCTATGGCCAGCTGGGG
AGCTTTTCCAACTGTTCTCACAGTAGGAATGACATGTGCCATTCTCTGGGGCTGA
CTTGTCTGGAGCCCCAGAAGACCACACCACCTACTACCCGACCACCACCTACAAC
TACCCCTGAACCAACCGCTCCACCACGACTGCAGCTGGTGGCACAGAGCGGAGG
ACAGCACTGTGCCGGAGTGGTCGAGTTCTACTCAGGCAGCCTGGGAGGCACCAT
CAGCTATGAGGCCCAGGACAAGACACAGGATCTGGAAAACTTCCTGTGCAACAA
TCTGCAGTGTGGCAGCTTTCTGAAACACCTGCCTGAGACAGAAGCAGGGAGGGC
ACAGGACCCAGGAGAGCCACGAGAACATCAGCCCCTGCCTATCCAGTGGAAAAT
TCAGAATTCAAGCTGCACTTCCCTGGAGCATTGTTTCCGAAAGATCAAACCACAG
AAGTCTGGACGGGTGCTGGCCCTGCTGTGCAGCGGATTTCAGCCCAAAGTGCAG
TCCAGGCTGGTCGGGGGATCCTCTATTTGCGAGGGGACAGTGGAAGTCCGCCAG
GGAGCTCAGTGGGCCGCCCTGTGCGATAGTTCAAGCGCACGGTCCTCTCTGAGAT

GGGAGGAAGTGTGCCGGGAACAGCAGTGTGGCAGTGTGAATTCATACAGAGTCC
TGGACGCTGGCGATCCCACCTCTAGGGGGCTGTTTTGTCCTCATCAGAAGCTGAG
TCAGTGTCACGAACTGTGGGAACGGAACTCATACTGTAAAAAGGTGTTTGTCACT
TGCCAGGATACAACTACGCCGGCGCCGAGACCACCTACACCTGCACCAACTATT
GCCTCTCAGCCACTGAGTCTGCGCCCCGAGGCATGTCGACCTGCCGCTGGCGGGG
CTGTGCACACCAGGGGCCTAGACTTCGCCTGCGATATCTATATTTGGGCTCCACT
GGCAGGAACCTGTGGCGTGCTGCTGCTGTCTCTGGTCATCACACTGTACTGCAAA
AGAGGCAGGAAGAAACTGCTGTATATTTTCAAGCAGCCCTTTATGAGACCTGTGC
AGACAACTCAGGAGGAAGACGGGTGCAGCTGTAGGTTCCCTGAGGAAGAGGAA
GGAGGCTGTGAGCTGCGCGTGAAATTTTCTCGGAGTGCAGATGCCCCAGCTTACC
AGCAGGGCCAGAACCAGCTGTATAACGAGCTGAATCTGGGGCGGAGAGAGGAA
TACGACGTGCTGGATAAGAGGCGCGGGCGAGATCCAGAAATGGGAGGAAAACC
CCAGCGACGGAAGAACCCTCAGGAGGGACTGTACAATGAACTGCAGAAGGACA
AAATGGCAGAGGCCTATTCCGAAATCGGGATGAAAGGAGAAAGAAGGCGCGGC
AAGGGGCATGATGGCCTGTATCAGGGACTGTCAACCGCAACAAAAGATACTTAT
GATGCTCTGCACATGCAGGCTCTGCCCCCGCGGTAA

SEQ ID NO: 15 is the nucleotide sequence which encodes CAR variant 2. SEQ ID NO: 15 is as follows:

ATGGCCCTGCCCGTGACCGGACTGCTGCTGAGCCTGGGCCTGCTGCTGCACGCCG
CTAGGCCAAGGCTGAGCTGGTACGATCCTGACTTTCAGGCCAGGCTGACCAGGT
CCAATTCCAAGTGTCAGGGCCAGCTGGAGGTGTACCTGAAGGACGGCTGGCACA
TGGAGACAGTGTGTTCCCAGAGCTGGGGCAGATCCAGCAAGCAGTGGGAGGACC
CTAGCCAGGCCTCCAAGGTGTGCCAGAGGCTGAATTGCGGCGTGCCCCTGAGCC
TGGGACCTTTCCTGGTGACCTACACCCCCCAGTCCAGCATCATCTGTTACGGCCA
GCTGGGCTCCTTTTCCAACTGCAGCCACTCCAGGAATGATATGGAGACCTGCCAC
AGCCTGGGCCTCACCTGTCTGGAGCCCCAGAAGACAACACCCCCTACCACAAGA
CCTCCCCCTACCACCACCCCTGAGCCCACCGCTCCCCCTAGACTGCAGCTGGTGG
CCCAGAGCGGCGGCCAACACTGCGCTGGAGTGGTGGAGTTCTACAGCGGCAGCC
TGGGCGGCACAATCAGCTACGAGGCCCAGGATAAGACCCAGGATCTGGAGAACT
TTCTGTGCAACAATCTGCAGTGTGGCAGCTTTCTGAAGCACCTGCCTGAGACAGA
GGCCGGCAGGGCCCAGGATCCTGGCGAACCAAGAGAGCACCAGCCCCTGCCTAT
CCAGTGGAAGATCCAGAATAGCTCCTGCACCTCCCTGGAGCACTGTTTTAGAAAG
ATCAAGCCCCAGAAGAGCGGCAGAGTGCTGGCCCTGCTGTGTAGCGGCTTCCAG

CCCAAGGTGCAGAGCAGGCTGGTGGGCGGCTCCTCCATCTGCGAGGGCACAGTG
GAGGTGAGACAGGGCGCCCAGTGGGCCGCTCTGTGTGATAGCAGCAGCGCCAGA
TCCAGCCTGAGGTGGGAGGAGGTGTGCAGGGAGCAGCAGTGTGGCTCCGTGAAC
TCCTACAGGGTGCTGGATGCCGGCGATCCTACATCCAGGGGCCTGTTTTGTCCCC
ACCAGAAGCTGAGCCAGTGCCACGAGCTGTGGGAGAGGAATTCCTACTGCAAGA
AGGTGTTCGTGACATGTCAGGACACAACCACCCCTGCCCCCAGACCTCCTACACC
CGCCCCTACCATCGCCTCCCAGCCCCTGAGCCTCAGGCCCGAAGCCTGTAGACCT
GCCGCCGGCGGAGCCGTTCACACCAGAGGACTGGATTTCGCCTGCGATATCTAC
ATCTGGGCCCCCCTGGCCGGCACCTGTGGAGTTCTGCTGCTGTCCCTGGTCATTA
CCCTGTACTGTAGAAGCAAGAGAAGCAGGCTGCTGCACTCCGATTACATGAATA
TGACCCCTAGAAGACCTGGCCCTACCAGGAAGCACTACCAGCCTTACGCCCCTCC
CAGGGACTTTGCCGCCTACAGATCCAAGAGAGGCAGAAAGAAGCTGCTGTACAT
CTTTAAGCAGCCTTTTATGGAGACAAGACCTGTGCAGACCACCCAGGAGGAGGA
CGGCTGCTCCTGCAGATTTCCTGAGGAGGAGGAGGGCGGCTGTGAGCTGAGGGT
GAAGTTTAGCAGGTCCGCCGATGCCCCTGCCTACCAGCAGGGCCAGAACCAGCT
GTACAATGAGCTGAACCTGGGCAGAAGGGAGGAGTACGACGTGCTGGATAAGA
GAAGGGGCAGGGATCCTGAGATGGAGACCGGCGGCAAGCCCCAGAGAAGGAAG
AATCCCCAGGAGGGCCTGTACAATGAACTGCAGAAGGACAAGATGGAGACAGC
CGAGGCCTACAGCGAGATCGGCATGGAGACCAAGGGCGAGAGAAGAAGGGGCA
AGGGCCACGACGGCCTGTACCAGGGCCTGAGCACAGCCACCAAGGATACATACG
ACGCCCTGCACATGCAGGCCCTGCCCCCTAGATGA

SEQ ID NO: 16 is the nucleotide sequence which encodes CAR variant 3. SEQ ID NO: 16 is as follows:

ATGGCCCTGCCCGTGACAGGCCTGCTGCTGAGCCTGGGCCTGCTGCTCCACGCCG
CTAGACCCAGACTGAGCTGGTACGATCCCGACTTTCAGGCCAGGCTGACAAGAA
GCAACAGCAAGTGTCAGGGCCAGCTGGAGGTGTACCTGAAGGATGGCTGGCACA
TGGAGACAGTGTGCAGCCAGTCCTGGGGCAGGTCCTCCAAGCAGTGGGAGGATC
CTTCCCAGGCCAGCAAGGTGTGTCAGAGACTGAATTGCGGCGTGCCCCTGAGCCT
GGGACCCTTTCTGGTGACATACACCCCTCAGAGCAGCATCATCTGCTACGGCCAG
CTGGGCTCCTTTAGCAATTGCTCCCACTCCAGGAATGATATGGAGACATGCCACA
GCCTGGGCCTCACCTGCCTGGAGCCCCAGAAGACCACACCCCCCACCACCAGGC
CCCCTCCTACCACAACCCCTGAGCCCACCGCCCCTCCCAGACTGCAGCTGGTGGC
CCAGAGCGGCGGCCAACACTGCGCTGGAGTGGTGGAGTTTTACAGCGGCAGCCT

GGGCGGCACAATCAGCTACGAGGCCCAGGACAAGACCCAGGACCTGGAGAATTT
TCTGTGTAATAATCTGCAGTGTGGCTCCTTCCTGAAGCACCTGCCCGAGACAGAG
GCCGGCAGAGCCCAGGACCCCGGAGAACCTAGGGAGCACCAGCCCCTGCCCATC
CAGTGGAAGATCCAGAACAGCTCCTGTACATCCCTGGAGCACTGCTTCAGGAAG
ATCAAGCCCCAGAAGTCCGGCAGAGTGCTGGCCCTGCTGTGTTCCGGCTTCCAGC
CCAAGGTGCAGTCCAGGCTGGTGGGCGGCTCCAGCATCTGCGAGGGCACAGTGG
AGGTGAGACAGGGCGCCCAGTGGGCCGCTCTGTGTGATAGCTCCAGCGCCAGAA
GCAGCCTGAGGTGGGAGGAGGTGTGCAGAGAGCAGCAGTGTGGCAGCGTGAATT
CCTACAGAGTGCTGGACGCCGGCGATCCTACAAGCAGGGGCCTGTTTTGTCCCCA
CCAGAAGCTGTCCCAGTGCCACGAGCTGTGGGAGAGAAATAGCTACTGTAAGAA
GGTGTTTGTGACCTGTCAGGACACAACCACCCCCGCCCCCAGGCCACCAACCCCT
GCTCCTACCATCGCCAGCCAGCCTCTGTCCCTGAGACCCGAGGCCTGTAGGCCTG
CCGCCGGAGGAGCTGTGCACACCAGAGGCCTGGATTTCGCCTGCGATATCCCCTT
CTTCTTTTGCTGCTTCATCGCCGTGGCCATGGGCATCAGATTCATCATCATGGTGG
CCATCTGGTGGAGGAGGAAGAGAAAGGAGAAGCAGTCCGAGACAAGCCCCAAG
GAGTTCCTGACAATCTACGAGGATGTGAAGGATCTGAAGACCAGGAGAAATCAC
GAGCAGGAGCAGACATTTCCTGGCGGCGGCAGCACCATCTACTCCATGATCCAG
TCCCAGTCCTCCGCCCCTACATCCCAGGAGCCCGCCTACACACTGTACTCCCTGA
TCCAGCCTAGCAGAAAGAGCGGCTCCAGAAAGAGAAACCACAGCCCTTCCTTCA
ACTCCACAATCTACGAAGTGATCGGCAAGAGCCAGCCCAAGGCCCAGAATCCCG
CCAGACTGAGCAGGAAGGAGCTGGAGAATTTCGATGTGTACTCCAGAGTGAAGT
TTTCCAGGAGCGCCGACGCCCCCGCCTACCAACAGGGACAGAATCAGCTGTACA
ATGAGCTGAACCTGGGCAGGAGGGAGGAGTACGACGTGCTGGACAAGAGAAGA
GGCAGGGACCCTGAGATGGGCGGCAAGCCTCAGAGAAGGAAGAATCCTCAGGA
GGGCCTGTACAACGAGCTGCAGAAGGACAAGATGGCCGAGGCCTACTCCGAGAT
CGGCATGAAGGGCGAGAGGAGAAGGGGCAAGGGCCACGACGGCCTGTACCAGG
GCCTGTCCACCGCCACCAAGGATACATACGATGCCCTGCACATGCAGGCCCTGCC
CCCCAGATGA

The third embodiment of the present invention refers to cells (hereinafter cells of the invention) comprising the above defined CAR of the invention.

In a preferred embodiment, the cell of the invention is a human T-cell or a human NK-cell.

In a preferred embodiment, the human T-cell is an autologous Tαβ-cell or an allogeneic Tγδ-cell, and the human NK-cell is an allogeneic cord blood-derived cell, a leukemic NK cell line-derived NK cell or an iPS-derived NK-cell.

The fourth embodiment of the present invention refers to a pharmaceutical composition (hereinafter pharmaceutical composition of the invention) comprising a plurality of cells of the invention and, optionally, a pharmaceutically acceptable carrier or diluents.

In a preferred embodiment, the pharmaceutical composition of the invention comprises a plurality of cells of the invention and, optionally, saline plus 2.5% human albumin as pharmaceutically acceptable carrier or diluent.

The fifth embodiment of the present invention refers to the pharmaceutical composition of the invention for use as a medicament, preferably for use in the treatment of invasive fungal infections, more preferably for use in the treatment of invasive fungal infections comprising fungal species from the genus *Candida, Aspergillus, Fusarium, Cryptococcus, Paracoccidiodes, Histoplasma, Rizhopus, Mucor* or *Arthrocladium.*

The sixth embodiment of the present invention refers to a method for treating invasive fungal infections, more preferably invasive fungal infections comprising fungal species from the genus *Candida, Aspergillus, Fusarium, Cryptococcus, Paracoccidiodes, Histoplasma, Rizhopus, Mucor* or *Arthrocladium,* which comprises the administration of a therapeutically effective amount of the pharmaceutical composition or the cells of the invention.

For the purpose of the present invention the following terms are defined:
- The term "comprising" means including, but not limited to, whatever follows the word "comprising". Thus, the use of the term "comprising" indicates that the listed elements are required or mandatory, but that other elements are optional and may or may not be present.
- By "consisting of" it is meant including, and limited to, whatever follows the expression "consisting of". Thus, the expression "consisting of" indicates that the listed elements are required or mandatory, and that no other elements may be present.
- "Pharmaceutically acceptable excipient or carrier" refers to a compound that may optionally be included in the compositions of the invention and that causes no significant adverse toxicological effects to the patient.
- By "therapeutically effective dose or amount" of a composition of the invention is intended an amount that, when administered as described herein, brings about a positive therapeutic response in a subject suffering from a fungal infection. The exact amount required will vary from subject to subject, depending on the age, the general condition of the subject, the severity of the condition being treated, the mode of administration, etc. An appropriate "effective" amount in any individual case may be determined by one of ordinary skill in the art using routine experimentation, based upon the information provided herein.

### Description of the figures

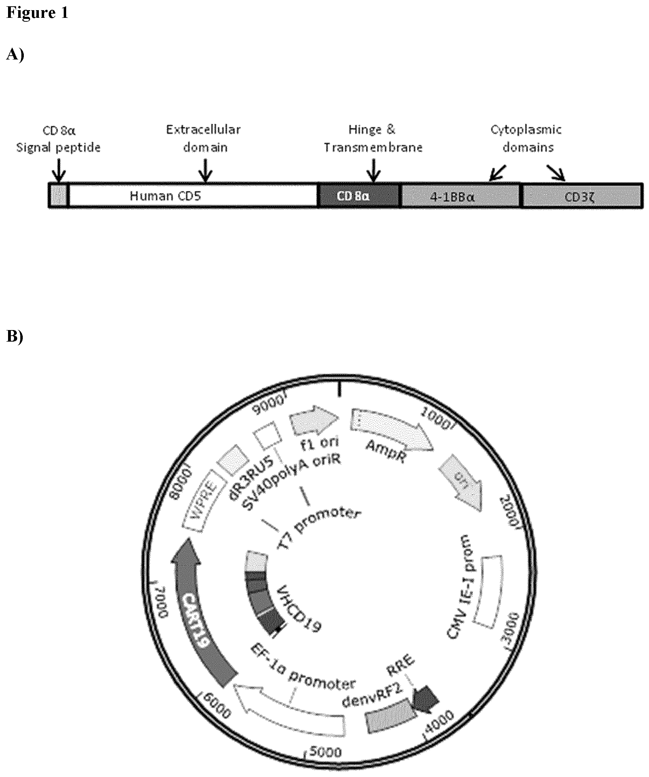

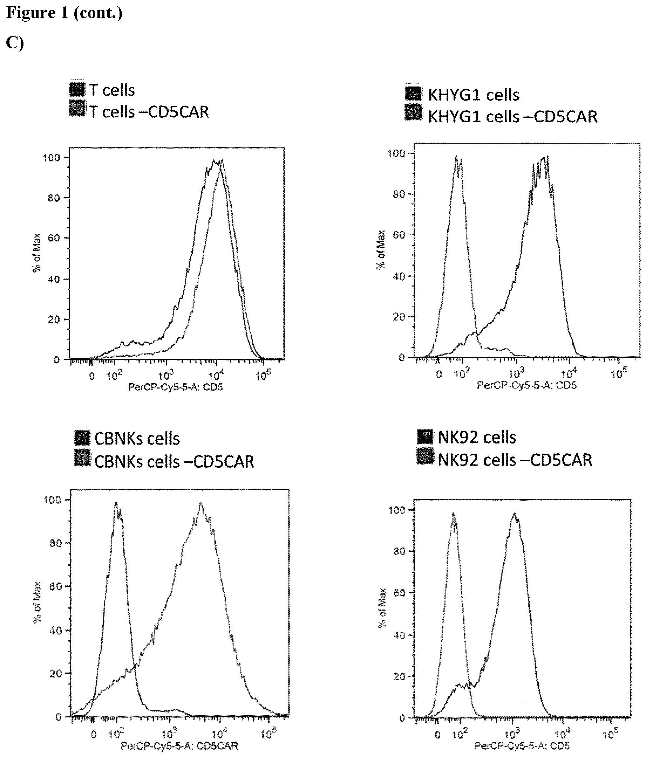

**Figure 1****. CD5CAR lentiviral vector design and expression. A)** Schematic representation of the CD5CAR construct (SEQ ID NO:11). **B)** Schematic map of the pCCLsinPPT EFla CART19 vector. The *Mlu*I and *BspE*I restriction sites used for CD5CAR construct insertion are indicated by arrows. **C)** CD5 expression on control (un-transduced) and CD5CAR-transduced human peripheral blood T cells, cord blood-derived NK cells (CBNK) and NK cell-lines (NK-92 and KHYG-1) by flow cytometry.

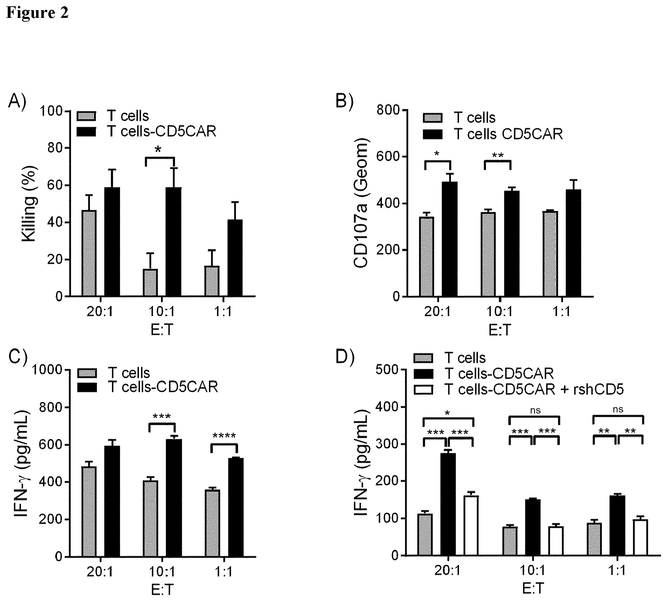

**Figure 2****. Effect of CD5CAR expression on human T cell-mediated antifungal response *ex vivo.* A)** CD5CAR-transduced and untransduced human peripheral blood T cells were co-cultured with alive C. *albicans* at the indicated E:T ratios for 4 h. Upon osmotic T-cell lysis, supernatants were seeded on Sabouraud dextrose agar plates for 48 h at 30 °C for CFUs counting. Results are represented as killing percentage over C. *albicans* culture without T cells. B) CD5CAR-transduced and untransduced T cells from 4 h-co-cultures as in A) were stained with anti-human CD107a for GeoMean analysis by flow cytometry. **C)** 24 h-supernatants from co-cultures of C. *albicans* with CD5CAR-transduced and untransduced human T cells were analysed for IFN-γ levels by ELISA. **D)** Same co-cultures as in C) in the presence or absence of rshCD5 (10 µg/mL). Statistical differences between groups were assessed by t-test or Mann Whitney test. *, p<0.05; **, p<0.01; ***, p<0.001; ****, p<0.0001.

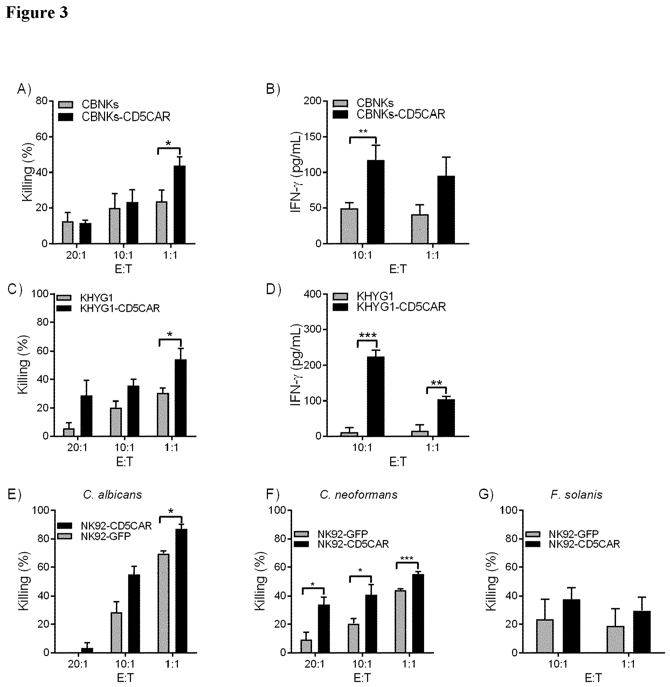

**Figure 3****. Effect of CD5CAR expression on human NK cell-mediated antifungal response *ex vivo.* A)** CD5CAR-transduced and untransduced cord blood-derived NK (CBNK) cells were co-cultured for 4 h with alive C. *albicans* at different indicated E:T ratios. Upon osmotic CBNK-cell lysis, culture supernatants were seeded on Sabouraud dextrose agar plates and incubated for 48 h at 30 °C for CFUs counting. Results are represented as killing percentage over C. *albicans* culture without T cells. **B)** Supernatants from 24 h-co-cultures of *C. albicans* with CD5CAR-transduced and untransduced CBNK cells were analysed for IFN-γ levels by ELISA. **C)** CD5CAR-transduced and untransduced KHYG-1 cells were co-cultured for 4 h with alive C. *albicans* at different indicated E:T ratios. Upon osmotic KHYG-1-cell lysis, culture supernatants were seeded on Sabouraud dextrose agar plates and incubated for 48 h at 30 °C for CFUs counting. Results are represented as killing percentage over C. *albicans* culture without KHYG-1 cells. **D)** Supernatants from 24 h-co-cultures of C. *albicans* with CD5CAR-transduced and untransduced KHYG-1 cells were analysed for IFN-γ levels by ELISA. **E-G)** CD5CAR-transduced and untransduced NK-92 cells were co-cultured for 4 h with alive C. *albicans, C. neoformas* or *F*. *solani* at different indicated E:T ratios. Upon osmotic NK-92-cell lysis, culture supernatants were seeded on Sabouraud dextrose agar plates and incubated for 48 h at 30 °C for CFUs counting. Results are represented as killing percentage over C. *albicans* culture without NK-92 cells. Statistical differences between groups were assessed by t-test or Mann Whitney test. *, p<0.05; **, p<0.01; ***, p<0.001.

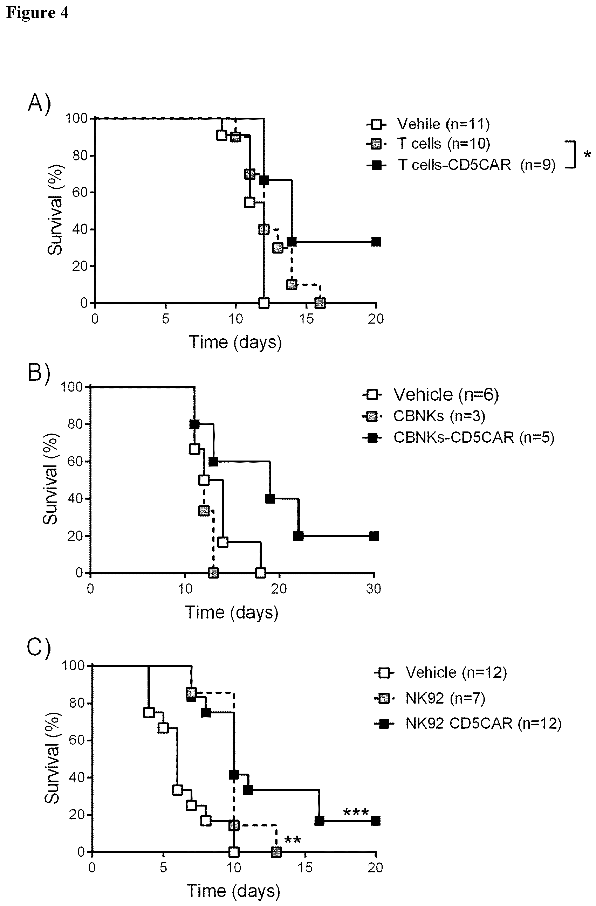

**Figure 4****. Effect of adoptively transferred human CD5CAR-T/NK cells on the survival of *C*. *albicans-infected* immunodeficient NSG mice. A)** Survival of C. *albicans-infected* (3 x 10² CFU/mouse i.v.) NSG mice treated with vehicle, CD5CAR-transduced or untransduced human T cells (4 x 10⁶ cells/mouse i.v.) 24 h post-infection was monitored overtime. **B)** Same as in A) treating C. *albicans-infected* NSG mice with vehicle, CD5CAR-transduced or untransduced CBNK cells (2 x10⁶ cells/mouse). **C)** Same as in A) treating C. *albicans-*infected NSG mice with vehicle, CD5CAR-transduced or untransduced uced NK-92 cells (2 x10⁶ cells/mouse). Statistical differences between groups were assessed by Log-rank (Mantel-Cox) Test. *, p<0.05; **, p<0.01; ***, p<0.001.

### Detailed description of the invention

The present invention is illustrated by means of the Examples set below without the intention of limiting its scope of protection.

### Example 1. Material and methods

### Example 1.1. Mice

Immunodeficient NSG (NOD/SCID IL-2Rγcnull) mice from Charles River Laboratories (France) were bred and kept in individual ventilated cages under specific pathogen-free (SPF) conditions. Unless otherwise stated, animals of 8 to 12 weeks of age were used in all experimental procedures, which were approved by the Animal Experimentation Ethical Committee of the University of Barcelona and Generalitat de Catalunya.

### Example 1.2. Generation and functional characterization of human lymphocytes expressing the CAR of the invention

### Example 1.2.1. CD5CAR construction

The CD5CAR-pCCL lentiviral expression vector of the invention was generated by replacing the CD19-scFv coding sequence from the third-generation lentiviral vector pCCLsinPPT_EF1a_CART19 vector (Milone *et al*., 2009; Porter *et al*., 2011; Castella *et al*., 2019) by SEQ ID NO: 11 as a *Mlu*I*-BspE*I fragment. The resulting CD5CAR construct is under the transcriptional control of the EF1α promoter and contains the coding sequences for the CD8α signal peptide (SEQ ID NO: 3), the CD5 extracellular sequence from position R25 to D369 (SEQ ID NO: 1), the CD8α hinge (SEQ ID NO: 4) and transmembrane region (SEQ ID NO: 5), and the intracytoplasmic activating motifs of the 4-1BB/CD137 (SEQ ID NO: 7) and CD3ζ (SEQ ID NO: 10) receptors. Alternative CD5CAR constructs (SEQ ID NO: 12 and SEQ ID NO: 13) were generated by combining the NKG2D transmembrane region (SEQ ID NO: 6), and the CD28 (SEQ ID NO: 8) and CD244/2B4 by (SEQ ID NO: 9) intracytoplasmic activating motifs.

### Example 1.2.2. Whole lentivirus particle production

HEK 293T cells (9 x 10⁶ cells) were plated into 10-cm culture dishes 24 h before co-transfection with the CD5CAR-pCCL vector and the packaging plasmids pRSV-Rev (Addgene, 12253), and pMDLg-pRRE(Addgene, 12251) and the envelope plasmid pMD2.G (Addgene, 12259). At the transfection time, 16 µg of total DNA (8 µg pCCL-CD5CAR, 4 µg pMDLg/pRRE, 2 µg pRSV-Rev, and 2 µg pMD2.G) were diluted in serum-free DMEM (41966-052; GIBCO) and then mixed with 40 µg of linear polyethylenimine (PEI) (23966-1; Polysciences) for 20 min at room temperature (RT). Next, medium was replaced, and DNA-PEI complexes were added into the cells. At 4 h post transfection, medium was replaced again. Complete lentiviral particles were collected from culture supernatants 72 and 96 h later, passed through 0.45 µm filters and concentrated by centrifugation at 18000 rpm for 3 h at 4 °C. Lentivirus-containing pellets were suspended in DMEM and stored at -80 °C until used. Whole lentiviral particles from packaging HEK 293T cells were used to transduce activated peripheral blood human T cells, cord blood-derived NK cells (CBNKs), or leukemia-derived NK cells (NK-92 or KHYG-1 cell lines), which were then subjected to different *in vitro* and *in vivo* experimental procedures.

### Example 1.2.3. Lentiviral transduction of T/NK cells and culture conditions

Buffy-coats and cord blood samples from healthy donors were provided by the Banc de Sang i Texits (BST) of Generalitat de Catalunya upon protocols approval by the internal Ethical Committee. For isolation of human T cells, buffy coats were subjected to density centrifugation over Ficoll (10771-6X100ML; Sigma-Aldrich) for isolation of peripheral blood mononuclear cells (PBMCs). Upon monocyte removal by adherence to plastic for 2 h at 37 °C in RPMI 1640 medium with L-glutamine supplemented with 10% FBS, 100 UI/mL penicillin (6191309; Lab EBN), and 100 µg/mL streptomycin (624569; Lab Normon), non-adherent PBMCs (1 x 10⁶ cells/mL) cells were then activated and expanded for 72 h with Dynabeads^{™} Human T-Activator CD3/CD28 (111-32D; GIBCO) (at a bead to cell ratio of 1:1) in the same RPMI 1640 medium as above further supplemented with 50 µM β-mercaptoethanol (31350-010; Thermo Fisher) and 30 IU/mL IL-2 (11011456001; Roche). For isolation of human CBNKs, cord blood samples were subjected to magnetic depletion with the NK cell Isolation Kit (MiltenyiBiotec, San Diego, CA) following the manufacturer's instructions. CBNK cells were then expanded for 14 days by co-culture with K562-based antigen presenting cells expressing membrane bound IL-21 (clone 9.mbIL21) in 45 % RPMI-1640 and 45 % Click's media (Irvine Scientific, Santa Ana, CA) plus 10 % human AB plasma (Atlanta Biologicals, Lawrenceville, GA) and IL-2 (400 IU/m added every other day; Proleukin, Chiron, Emeryville, CA).

The human NK cell line NK-92 was obtained from American Type Culture Collection (ATCC, CRL-2407). The human NK cell line KHYG1 was kindly provided by Michael O'Dwyer (ONK Therapeutics, Galway, Ireland). NK-92 cells were cultured in X-VIVO 10 medium (H3BEBP04-743Q, Lonza) supplemented with 5% human AB plasma and 10 ng/mL IL-2 (200-02, Peprotech). KHYG-1 cells were cultured in RMPI 1640 medium with L-glutamine and supplemented with 10% FBS, 100 UI/mL penicillin, 100 µg/mL streptomycin and 10 ng/mL IL-2 (200-02, Peprotech).

Lentiviral transduction of T, CBNK, NK-92 or KHYG-1 cells (1 x 10⁶) was performed by centrifugation (2000 rpm) for 90 min at 32 °C followed by 5 h-incubation at 37 °C in a 5 % CO₂ atmosphere, in the presence of 4 µg/mL of polybrene (TR-1003-G; Merck Millipore) in the corresponding growth media for each cell type. Next, fresh medium was added to avoid polybrene toxicity and left in culture for 72 h for further cell surface CD5CAR expression analysis by flow cytometry. To this end, CD5CAR-transduced and untransduced cell controls were adjusted to the desired concentration (1 x 10⁶ cells/mL) in staining solution (PBS plus 2% FBS) plus PercPCy5.5-labelled anti-human CD5 monoclonal antibody (mAb, clone UCHT2; TONBO) and incubated for 20 min at 4 °C in dark. Next, cells were centrifuged at 1500 r.p.m., washed twice with PBS and fixed in PBS plus 1% paraformaldehyde (PFA) for analysis in a BD FACSCanto II flow cytometer. Mean fluorescence intensity (MFI) or percentage of positive cells was assessed using FlowJo software (Tree Star, USA).

### Example 1.2.4. In vitro assays of antifungal activity

All functional assays with CD5CAR-T/NK cells and the corresponding untransduced cells were performed at 72 h post lentiviral transduction. CD5CAR-transduced and untransduced T/NK cells were co-cultured for 4 h at 37 °C in a 5 % CO₂ atmosphere with alive C. *albicans, C. neoformans* or *F*. *solani* at different effector:target (E:T) ratios. Next, cells were subjected to osmotic lysis and supernatants seeded on Sabouraud dextrose agar plates for 48 h at 30 °C for CFUs counting. Killing percentage was calculated with regard to fungal cells cultured alone. In parallel experiments, the expression of the T/NK cell degranulation marker CD107a/LAMP1 was assessed by staining with PE-labelled H4A3 mAb (BD Pharmigen) and further analysis of MFI or percentage of positive cells by flow cytometry. The concentration of IFN-γ in supernatants from 24 h co-cultures (in the presence or absence of recombinant soluble human CD5 protein; 10 µg/mL) was assessed by ELISA (BD OptEIA-Human ELISA Set) following the manufacturer's instructions.

### Example 1.3. In vivo analysis of adoptive CD5CAR-T/NK cell transfer into fungal-infected immunodeficient NSG mice.

### Example 1.3.1 Mouse model of fungal infection by C. albicans

*C. albicans* (strain SC5314; ATCC MYA-2876), kindly provided by Dr. Oscar Zaragoza (Instituto de Salud Carlos III, Madrid), was grown for 48 h at 30 °C on Sabouraud agar plates (01024_00; Conda). Then, a single colony was grown overnight (o/n) at 37 °C in Sabouraud liquid medium (CM0147; Oxoid) under horizontal shaking at 180 rpm. The desired concentration for inoculum preparation was achieved after plating serial dilutions in saline. Infection of NSG mice was performed by intravenous (i.v.; tail vein) injection of 3 x 10⁴ - 3 x 10² colony forming units (CFUs) per gram. Survival and body weight loss were monitored daily.

### Example 2. Results

### Example 2.1. Development of CD5-based CAR for adoptive T/NK cell transfer therapies

With the aim of developing a therapeutic strategy against systemic fungal infections, human T and NK cells expressing an activating CD5-based chimerical receptor (CD5CAR) were generated for further adoptive cell transfer therapeutic purposes. To this end, a second generation CD5CAR construction was designed composed of the CD8α signal peptide, the human CD5 extracellular ectodomain (from Arg25 to Asp369), the CD8α transmembrane region and the intracytoplasmic activating domains of 4-1BB/CD137 and CD3ζ receptors **(****Figure 1B**). The CD5CAR construct was cloned into a modified version of the third-generation lentiviral vector pCCL as a *Mlu*I*-BspE*I fragment under the transcriptional control of the EF-1α promoter **(****Figure 1B**). CD5CAR expression was achieved by transduction of human T and NK cells with whole lentiviral particles from packaging HEK 293T cells **(****Figure 1C****),** which were then subjected to different *in vitro* and *in vivo* experimental procedures.

### Example 2.2. In vitro analysis of anti-fungal responses from CD5CAR-T/NK cells

CD5CAR-transduced peripheral blood human T cells were co-cultured for 4 h with alive C. *albicans* conidia at different effector:target (E:T) ratios. As illustrated by **Figure 2****,** CD5CAR-T cells showed higher percentage of C. *albicans* killing **(****Figure 2A****),** higher surface levels of the LAMP-1/CD107a degranulation marker **(****Figure 2B****),** and higher IFN-γ production levels **(****Figure 2C****),** at all the E:T ratios tested, compared to untransduced T cells. Moreover, the differences between CD5CAR-T and untransduced T cells regarding IFN-γ production were abrogated by the addition of rshCD5 (10 µg/mL) to the co-cultures **(****Figure 2D****),** thus confirming the CD5-mediated specificity of the phenomenon.

Similar results regarding higher percentage of C. *albicans* killing and IFN-g production were observed when CD5CAR-CBNK cells were co-cultured for 4 h with alive C. *albicans* at different E:T ratios **(****Figure 2E and 2F****).**

Fungal killing experiments in which alive C. *albicans, C. neoformans* and *F*. *solani* were co-cultured for 4 h with CD5CAR-transduced or untransduced NK-92 cells at different E:T ratios demonstrated the higher killing capability of the former cells against different fungal species, thus confirming their broad anti-fungal activity **(****Figure 2G, 2H and 2I****)**

### Example 2.3. In vivo efficacy of adoptive CD5CAR-T/NK cell transfer therapy in fungal-infected immunodeficient NSG mice

The *in vivo* efficacy of adoptively transferred CD5CAR-T cells and untransduced human T cells to C. *albicans*-infected immunodeficient NSG mice was next investigated. To that end, a lethal inoculum of C. *albicans* (8x10³ CFU/mouse/gr; as determined in previous dose-course experiments) was i.v. injected to NSG mice. Then, mice were adoptively transferred with CD5CAR-T or untransduced human T cells (4 x 10⁶ cells/mouse, i.v.) at 24 h post-infection. As illustrated by **Figure 3A****,** CD5CAR-T cells induced higher survival rates than observed in control groups (vehicle and untransduced T cells).

Similar in vivo efficacy results were observed when C. *albicans*-infected NSG mice were adoptively transferred at 24 h post-infection with CD5CAR-CBNK (4 x 10⁶ cells/mouse, i.v.) and CD5CAR-NK92, (4 x 10⁶ cells/mouse, i.v.) compared to their untransduced cell controls **(****Figure 4B and 4C****).**

## Claims

1. A chimeric antigen receptor (CAR) comprising an extracellular domain which in turn comprises SEQ ID NO: 1.

2. CAR, according to claim 1, comprising an extracellular domain which consists of SEQ ID NO: 1 or SEQ ID NO: 2.

3. CAR, according to any of the claims 1 or 2, further comprising a signal peptide, a transmembrane domain, and one or more intracellular activation domains.

4. CAR, according to any of the previous claims, wherein the signal peptide consist of CD8α of SEQ ID NO: 3, the hinge domain consists of CD8α of SEQ ID NO: 4, the transmembrane domain consists of CD8α of SEQ ID NO: 5 or NKGD2 of SEQ ID NO: 6, and the intracellular activation domain consists of CD137/4-1BB of SEQ ID NO: 7, CD28 of SEQ ID NO: 8, CD244/2B4 of SEQ ID NO: 9 and/or of CD3ζ (zeta) of SEQ ID NO: 10.

5. CAR, according to any of the previous claims, comprising SEQ ID NO: 11, SEQ ID NO: 12 or SEQ ID NO: 13.

6. A nucleic acid encoding the CAR according to any of the claims 1 to 5.

7. A nucleic acid, according to claim 6, comprising SEQ ID NO: 14, SEQ ID NO: 15 or SEQ ID NO: 16.

8. A cell comprising the CAR of any of the claims 1 to 5 or the nucleic acid according to any of the claims 6 or 7.

9. A cell, according to claim 8, **characterized in that** it is a T-cell or a NK-cell.

10. A cell, according to any of the claims 8 or 9, **characterized in that** the T-cell is an autologous Tαβ-cell or an allogeneic Tγδ-cell, and the NK-cell is an allogeneic cord blood-derived, leukemic cell line-derived NK-cell or an iPS-derived NK-cell.

11. A pharmaceutical composition comprising a plurality of cells according to any of the claims 8 to 10 and, optionally, a pharmaceutically acceptable carrier or diluents.

12. A pharmaceutical composition comprising a plurality of cells, according to claim 11 and, optionally, saline plus 2.5% human albumin as pharmaceutically acceptable carrier or diluent.

13. A cell, according to any of the claims 8 to 10, or the pharmaceutical composition according to claim 11 or 12, for use as a medicament.

14. A cell or pharmaceutical composition, according to claim 13, for use in the treatment of fungal infections.

15. A cell or pharmaceutical composition, according to any of the claims 13 or 14, for use in the treatment of invasive fungal infections caused by fungal species comprising the genus *Candida, Aspergillus, Fusarium, Cryptococcus, Paracoccidiodes, Histoplasma, Rizhopus, Mucor* or *Arthrocladium.*
